# EUROPEAN PATENT APPLICATION

(11) **EP 4 091 528 A1**
(43) Date of publication of application: **23.11.2022**
(21) Application number: 22174396.6
(22) Date of filing: 19.05.2022
(51) Int. Cl.: A61B 1/00, G06T 7/00

(54) **ENDOSCOPE SYSTEM AND METHOD FOR OPERATING THE SAME**

(30) Priority: 21.05.2021 JP 2021086549
(71) Applicant: FUJI-FILM Corporation, Minato-ku Tokyo 106-8620 (JP); Fujita Academy, Toyoake-shi, Aichi 470-1192 (JP)
(72) Inventor: TERAMURA, Yuichi, Kanagawa (JP); OTAKA, Yohei, Aichi (JP); KAGAYA, Hitoshi, Aichi (JP); SHIBATA, Seiko, Aichi (JP)
(74) Representative: Hughes, Andrea Michelle

(57) **Abstract**

Provided are an endoscopy system and a method for operating the endoscopy system which reduce a burden of observing an image obtained during endoscopy.

An endoscope system (10) that illuminates an object and captures reflected light from the object includes a control processor (15). The control processor acquires an examination image and determines whether the examination image shows a swallowing state or a non-swallowing state. In addition, the control processor detects a high pixel value region from the examination image and determines that the examination image shows the swallowing state in a case in which an area of the high pixel value region is equal to or greater than a first threshold value. Further, the control processor performs grayscale conversion on the examination image to obtain a grayscale image and performs a binarization process for obtaining the high pixel value region in a case in which a density value of a pixel of the grayscale image is equal to or greater than a second threshold value.

## Description

### BACKGROUND OF THE INVENTION

### 1. Field of the Invention

The present invention relates to an endoscope system having a unit for analyzing an image obtained by an examination and a method for operating the same.

### 2. Description of the Related Art

A state in which it is difficult to swallow food or drink is referred to as swallowing disorder. It is said that, in a case in which the swallowing disorder occurs, it is more likely to cause suffocation or aspiration pneumonia due to accidental influx of food into the airways. Since the swallowing disorder occurs with aging or due to nervous system diseases, it becomes more and more important to examine a swallowing function in the aging society in recent years. The swallowing function is examined to specify the condition of aspiration and to appropriately treat and prevent the swallowing disorder.

Several new methods are being developed to examine the swallowing function. For example, in JP2016-185209A, speech waveform data including swallowing sounds is acquired, and sounds associated with swallowing, such as an epiglottis closing sound, an esophageal passage sound, and an epiglottis opening sound, are analyzed in detail. Further, in WO2018/193955A, the movement of the skin or surface muscles or a distance between the corners of the mouth is measured and analyzed by a three-dimensional shape measurement device to evaluate the swallowing function.

### SUMMARY OF THE INVENTION

The measurement methods described in JP2016-185209A and WO2018/193955A are relatively new methods. Video fluoroscopic examination of swallowing (VF) using X-rays and video endoscopic examination of swallowing (VE) have been clinically established as swallowing disorder evaluation methods (swallowing function evaluation examination). The video fluoroscopic examination of swallowing is an examination that causes a subject to swallow a contrast medium and obtains radiographic images of the pharynx, the larynx, and the esophagus during swallowing. The video endoscopic examination of swallowing is an examination that inserts an endoscope into the body through the nose and obtains endoscopic images of the pharynx and the larynx, particularly, the vicinity of the epiglottis during swallowing. In the video endoscopic examination of swallowing, it is necessary to observe a large number of images acquired. Therefore, there is a possibility that oversight will occur during the examination. In addition, it is a burden on the user to review moving images for a long time after the examination. Therefore, there is a demand for a technique for reducing the burden on the user who observes.

An object of the present invention is to provide an endoscopic system and a method for operating the endoscopic system which reduce a burden of observing an image obtained during endoscopy.

According to an aspect of the invention, there is provided an endoscope system that illuminates an object and captures light from the object. The endoscope system comprises a control processor. The control processor acquires an examination image and determines whether the examination image shows a swallowing state or a non-swallowing state.

Preferably, the control processor detects a high pixel value region from the examination image and determines that the examination image shows the swallowing state in a case in which an area of the high pixel value region is equal to or greater than a first threshold value.

Preferably, the control processor performs grayscale conversion on the examination image to obtain a grayscale image and performs a binarization process for obtaining the high pixel value region in a case in which a density value of a pixel of the grayscale image is equal to or greater than a second threshold value.

Preferably, the control processor decides a region to be determined from the examination image and detects the high pixel value region from the region to be determined. Preferably, the region to be determined is a region in a range which has at least 10 pixels or more from an image center of the examination image in a vertical direction and a horizontal direction and in which a size of one side of the region to be determined is equal to or less than half a size of the smaller of vertical and horizontal sides of the examination image. Preferably, the control processor detects an epiglottis region from the examination image and uses the epiglottis region as the region to be determined.

Preferably, the control processor inputs the examination image to a classifier and outputs the examination image determined to show the swallowing state or the non-swallowing state. Preferably, the classifier is trained with an image determined to show the swallowing state or the non-swallowing state.

Preferably, after acquiring the examination image determined to show the swallowing state, the control processor determines that the examination images of frames acquired for a predetermined period show the swallowing state and outputs the examination images acquired for the predetermined period as a swallowing moving image. Preferably, the predetermined period is settable to any value. Preferably, the predetermined period is automatically set on the basis of a time required for a swallowing movement.

According to another aspect of the invention, there is provided a method for operating an endoscope system that illuminates an object, captures light from the object, and includes a control processor. The method comprises: a step of causing the control processor to acquire an examination image; and a step of causing the control processor to determine whether the examination image shows a swallowing state or a non-swallowing state.

According to the endoscope system and the method for operating the endoscope system of the invention, it is possible to provide an endoscope system and a method for operating the endoscope system that reduce a burden of observing an image obtained during endoscopy.

### BRIEF DESCRIPTION OF THE DRAWINGS

Fig. 1 is a block diagram illustrating an endoscope system.
Fig. 2 is a graph illustrating a spectrum of normal light.
Fig. 3 is a block diagram illustrating a function of a swallowing determination unit.
Fig. 4 is a diagram illustrating swallowing.
Fig. 5 is a diagram illustrating aspiration.
Fig. 6 is a diagram and an image diagram illustrating a method for capturing an examination image.
Fig. 7 is a diagram illustrating a method for determining swallowing in a first determination unit.
Fig. 8 is a diagram illustrating a method for selecting a region to be determined from a size from an image center and the size of the examination image.
Fig. 9 is a diagram illustrating a method for detecting an epiglottis region and using the epiglottis region as the region to be determined.
Fig. 10 is a diagram illustrating a method for determining swallowing in a second determination unit.
Fig. 11 is a diagram illustrating a method for creating a swallowing moving image.

### DESCRIPTION OF THE PREFERRED EMBODIMENTS

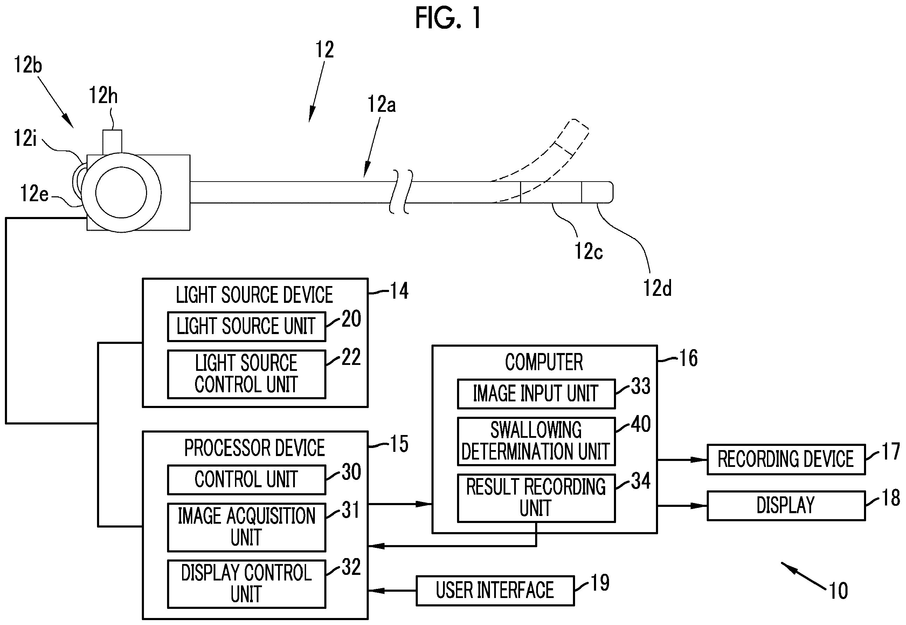

As illustrated in Fig. 1, an endoscope system 10 comprises an endoscope 12, a light source device 14, a processor device 15, a computer 16, a recording device 17, a display 18, and a user interface 19. The endoscope 12 is optically connected to the light source device 14 and is electrically connected to the processor device 15. The endoscope 12 has an insertion portion 12a that is inserted into a body to be observed, an operation portion 12b that is provided in a base end portion of the insertion portion 12a, and a bending portion 12c and a tip portion 12d that are provided on the tip side of the insertion portion 12a. The bending portion 12c is bent by operating an angle knob 12e of the operation portion 12b. The bending portion 12c is bent to move the tip portion 12d in a desired direction. The endoscope 12 may be a fiberscope or may be located at an operation portion side end of the insertion portion 12a. The endoscope 12 is an endoscope that is used for swallowing endoscopy.

An optical system for forming an object image and an optical system for irradiating an object with illumination light are provided inside the endoscope 12. The object is a structure in a living body related to swallowing movement. Specifically, the object is the pharynx and the larynx. The operation portion 12b is provided with a still image acquisition instruction switch 12h that is used to input an instruction to acquire a still image of the object to be observed and a zoom operation portion 12i that is used to operate a zoom lens, in addition to the angle knob 12e.

The light source device 14 generates illumination light. For example, the processor device 15 controls the endoscope system 10 and performs image processing on an image signal output from the endoscope 12. The display 18 is a display unit that displays an image captured by the endoscope 12. The user interface 19 is an input device that inputs settings and the like to the processor device 15 and the like.

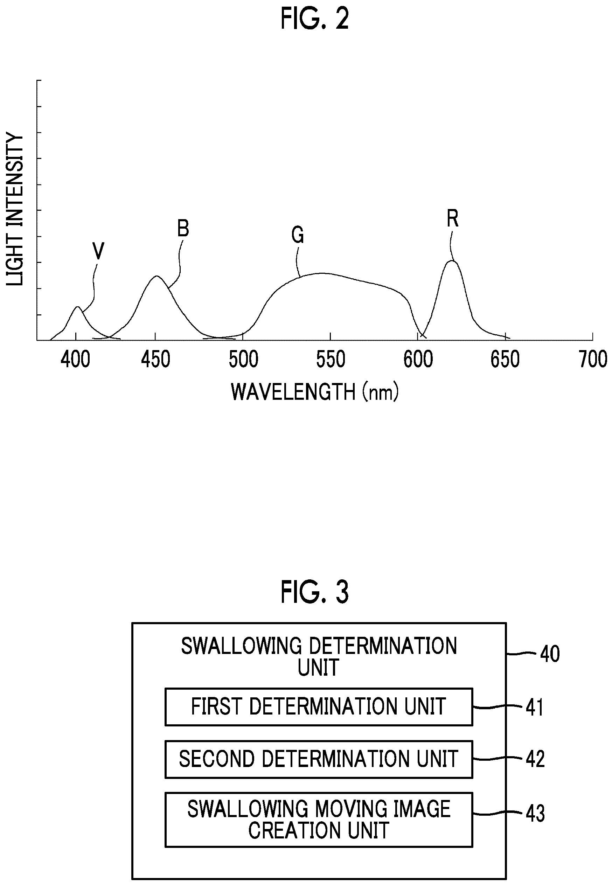

The light source device 14 comprises a light source unit 20 that emits the illumination light and a light source control unit 22 that controls the operation of the light source unit 20. The light source unit 20 emits the illumination light for illuminating the object. The light source unit 20 includes a light source, such as a laser diode, a light emitting diode (LED), a xenon lamp, or a halogen lamp, and emits at least illumination light (normal light) having a spectrum illustrated in Fig. 2. In addition, the light source unit 20 may be provided in the endoscope 12. In this case, the light source unit 20, the endoscope 12, and the processor device 15 are wirelessly connected. Further, the light source control unit may be provided in the endoscope 12 or may be provided in the processor device 15. White includes so-called pseudo-white which is substantially equivalent to white in the imaging of the object by the endoscope 12 is mixed with purple light V, blue light B, green light G, or red light R as illustrated in Fig. 2. Furthermore, the light source unit 20 includes, for example, an optical filter that adjusts the wavelength band, spectrum, or amount of the illumination light, if necessary.

The light source control unit 22 controls, for example, the turn-on or turn-off of each light source constituting the light source unit 20 and the amount of light emitted from each light source. An illumination optical system and an imaging optical system are provided in the tip portion 12d of the endoscope 12. The illumination light emitted by the light source unit 20 passes through the insertion portion 12a of the endoscope 12 through a light guide and is emitted from the tip portion 12d to the object through an illumination lens of the illumination optical system. In addition, in a case in which the light source unit 20 is provided in the tip portion 12d of the endoscope, the illumination light is emitted to the object through the illumination lens of the illumination optical system without passing through the light guide. The imaging optical system includes an objective lens and an imaging sensor. Light reflected from the object to be observed by the emission of the illumination light is incident on the imaging sensor through the objective lens and the zoom lens. Therefore, an image of the object to be observed is formed on the imaging sensor. The zoom lens is a lens for enlarging the object to be observed and is moved between a telephoto end and a wide end by the operation of the zoom operation portion 12i.

Examples of the imaging sensor include a complementary metal oxide semiconductor (CMOS) sensor and a charge-coupled device (CCD) sensor. An examination image is generated on the basis of an image signal detected by the imaging sensor.

The imaging sensor may include a color filter provided with a color filter (for example, a Bayer filter) that converts the sensed light into a color image signal and a monochrome image sensor that is not provided with a color filter converting the sensed light into a monochrome image signal. In addition, the color image sensor may be a sensor that does not convert the sensed light into an RBG signal, but converts the sensed light into a CMY signal.

In a case in which a color image is acquired, the image signal includes a B image signal output from a B pixel, a G image signal output from a G pixel, and an R image signal output from an R pixel. The image signal is output to an image acquisition unit 31 of the processor device 15 and is acquired as an examination image which is a monochrome image or a color image. The examination image acquired by the image acquisition unit 31 is output to an image input unit 33 of the computer 16. The examination image output to the image input unit 33 is output to a swallowing determination unit 40. The examination images are a series of moving images which are captured during endoscopy and are continuous in time series.

The processor device 15 includes a control unit 30, the image acquisition unit 31, and a display control unit 32. In the processor device 15, the control unit 30 composed of a control processor operates a program in a program memory to implement the functions of the image acquisition unit 31 and the display control unit 32.

The computer 16 includes the image input unit 33, the swallowing determination unit 40, and a result recording unit 34. In the computer 16, a central control unit (not illustrated) composed of a control processor operates a program in the program memory to implement the functions of the image input unit 33, the swallowing determination unit 40, and the result recording unit 34. In addition, the computer 16 and/or the light source control unit 22 may be included in the processor device 15. The result recording unit 34 records the time when the swallowing movement is performed and the number of times the swallowing movement is performed, generates an image to be displayed on the display 18 or an image to be output to the recording device 17, and edits the moving image.

Hereinafter, the function of the swallowing determination unit 40 will be described with reference to Fig. 3. The swallowing determination unit 40 comprises a first determination unit 41, a second determination unit 42, and a swallowing moving image creation unit 43. The swallowing determination unit 40 determines whether the acquired examination image shows a swallowing state or a non-swallowing state and extracts a moving image (swallowing moving image) determined to show the swallowing state from the moving images obtained by the examination.

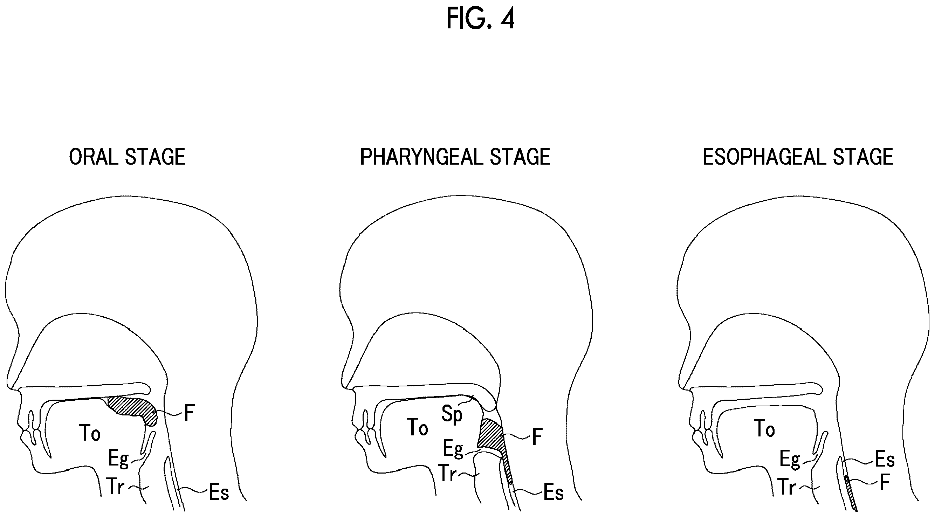

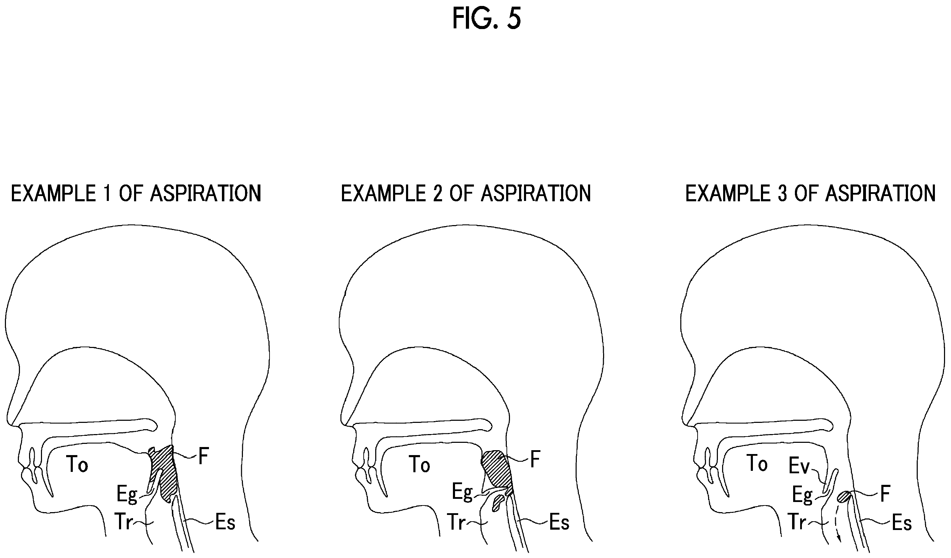

The term "swallowing" means a series of actions which put food or drink in the mouth, chew and swallow it, and transport it to the esophagus. Fig. 4 is a diagram illustrating normal swallowing, and Fig. 5 is a diagram illustrating abnormal swallowing (aspiration). As illustrated in Fig. 4, the swallowing movement is mainly divided into an "oral stage" in which food F is mainly transported from the oral cavity to the pharynx by the movement of a tongue To, a "pharyngeal stage" in which the food F is transported from the pharynx to the esophagus Es by the swallowing reflex, and an "esophageal stage" in which the food F is transported from the esophagus Es to the stomach by the peristaltic movement of the esophagus. At the time of swallowing, the food F is directed toward the esophagus Es and does not flow into the tracheal Tr. Therefore, the epiglottis Eg, which plays a role of covering the tracheal Tr, closes the entrance (glottis) of the tracheal Tr by the reflex movement. In addition, the soft palate Sp, which is the ceiling of the oral cavity, also moves backward to close the passage between the oral cavity and the nasal cavity such that the food F does not enter the nasal cavity. In a case in which any dysfunction occurs at any of the oral stage, the pharyngeal stage, or the esophageal stage, as illustrated in Fig. 5, the food F that should be transported to the esophagus Es in a normal state flows into the trachea Tr, which is called aspiration.

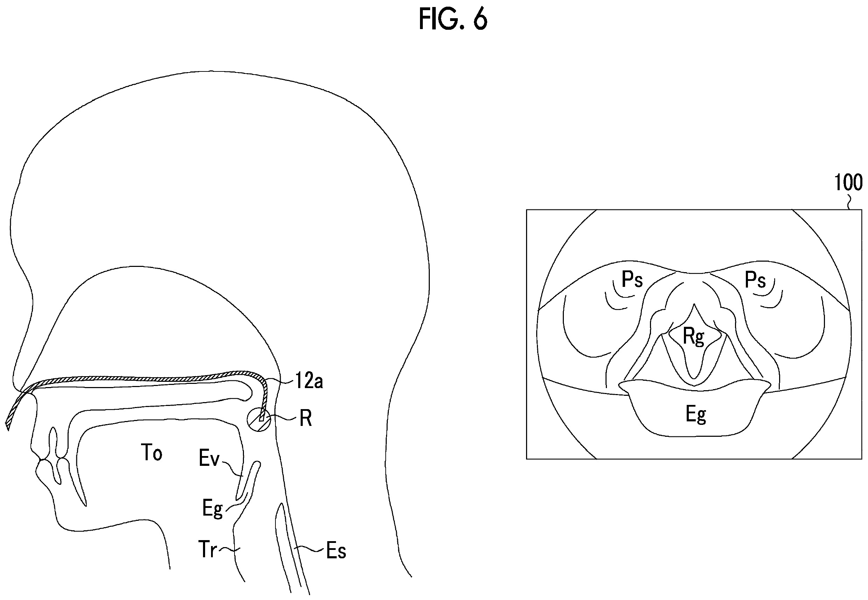

Example 1 of the aspiration illustrated in Fig. 5 is an example of aspiration in which the food F flows into the trachea Tr from the oral stage to the pharyngeal stage before the swallowing reflex occurs. Example 2 of the aspiration illustrated in Fig. 5 is an example of aspiration in which the food F flows into the trachea Tr due to the incomplete closure of the glottis (the entrance of the trachea Tr) by the epiglottis Eg in the middle of the swallowing reflex. Example 3 of the aspiration illustrated in Fig. 5 is an example of aspiration in which the food F remaining in the epiglottic vallecula Ev or the pyriform sinuses (see an example 100 of an examination image in Fig. 6), which are depressions present on the left and right sides of the entrance of the esophagus, flows into the trachea Tr after the swallowing reflex.

The examination image acquired in this embodiment is captured by inserting the insertion portion 12a of the endoscope 12 from the nasal cavity into the pharynx such that the tip portion 12d of the endoscope is located near a position R of the oropharynx illustrated in Fig. 6. As illustrated in the example 100 of the examination image of Fig. 6, it is preferable that the examination image includes anatomical structures such as the epiglottis Eg, the rima glottidis Rg, and the left and right pyriform sinuses Ps. The rima glottidis Rg is a space between the left and right folds constituting the vocal cords. A case in which the tip of the endoscope is disposed in the oropharynx will be described below. However, the tip of the endoscope may be disposed in the rhinopharynx, the epipharynx, the hypopharynx, or the larynx, in addition to the oropharynx, to determine the swallowing.

Either the first determination unit 41 or the second determination unit 42 determines the swallowing. In addition, the swallowing may be determined by combining the determination results of the first determination unit 41 and the second determination unit 42 in order to improve the accuracy of the determination. Analysis performed by the swallowing determination unit 40 will be described below.

The first determination unit 41 determines whether the examination image shows the swallowing state or the non-swallowing state on the basis of the area of a high pixel value region in the examination image. The high pixel value region is a region having a pixel value equal to or greater than a predetermined value and is, specifically, a region in which halation, overexposure, or whiteout has occurred. First, the examination image output from the image input unit 33 is grayscale-converted into a grayscale image. For example, gamma correction is used for the grayscale conversion. Then, the grayscale image is binarized to generate a binarized image, and the binarized image is divided into a high pixel value region and a low pixel value region. Here, in a case in which the area of the high pixel value region in the examination image is equal to or greater than a first threshold value, it is determined that the examination image shows the swallowing state.

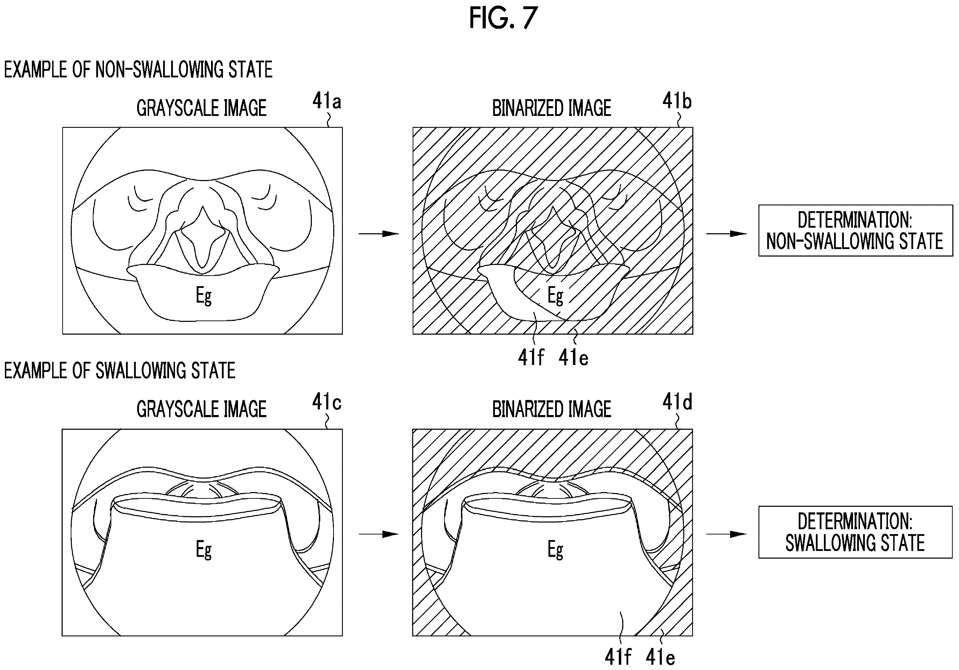

Fig. 7 illustrates a specific example of the determination of the swallowing by the first determination unit 41. An upper part of Fig. 7 illustrates an example using the examination image showing the non-swallowing state, and a lower part of Fig. 7 illustrates an example using the examination image showing the swallowing state. First, the examination image is grayscale-converted into a grayscale image (a grayscale image 41a in the upper part of Fig. 7 and a grayscale image 41c in the lower part of Fig. 7). Then, the grayscale image is binarized to obtain a binarized image (a binarized image 41b in the upper part of Fig. 7 and a binarized image 41d in the lower part of Fig. 7). In the binarized images 41b and 41d illustrated in Fig. 7, it is assumed that a hatched portion is a low pixel value region 41e and a white portion is a high pixel value region 41f. Here, in a case in which the area of the high pixel value region 41f is equal to or greater than the first threshold value, it is determined that the image shows the swallowing state. In a case in which the area of the high pixel value region 41f is less than the first threshold value, it is determined that the image shows the non-swallowing image. In Fig. 7, the binarized image 41b is determined to show the non-swallowing state, and the binarized image 41d is determined to show the swallowing state.

During swallowing, for example, the soft palate Sp, the tongue To, and the epiglottis Eg move violently and contract with the swallowing movement to cover the tip of the endoscope. The determination of the swallowing by the first determination unit 41 uses the fact that, during swallowing, the surrounding tissues cover the front side of the illumination light emitting unit and the image sensor of the endoscope 12 such that automatic exposure control does not work and an overexposed region increases. In addition, the first threshold value can be set to any value.

In the process of binarizing the grayscale image, it is preferable that a threshold value of a density value for dividing the binarized image into the high pixel value region and the low pixel value region is set as a second threshold value. In a case in which the density value of each pixel of the grayscale image is equal to or greater than the second threshold value, the region is defined as the high pixel value region. In a case in which the density value is less than the second threshold value, the region is defined as the low pixel value region.

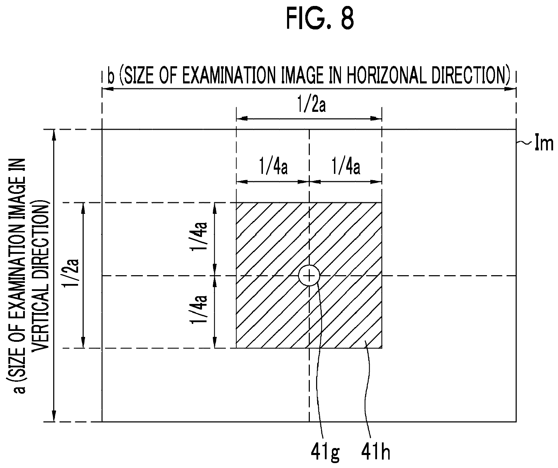

Further, in a case in which the grayscale conversion or the binarization process is performed, the region to be determined may be decided from the examination image, and the grayscale conversion or the binarization process may be performed only on the region to be determined to detect the high pixel value region. Then, the swallowing determination may be performed. The range of the region to be determined is, for example, a range which is at least 10 pixels or more from an image center 41g of an examination image Im of each frame in the vertical and horizontal directions and in which the size of one side of the region to be determined is equal to or less than half the size of the smaller of the vertical and horizontal sides of the examination image. In a specific example illustrated in Fig. 8, the vertical size of the examination image Im is "a" pixels, the horizontal size thereof is "b" pixels, and a < b is satisfied. A region 41h to be determined is a region having a width of 1/4a pixels from the image center in the vertical and horizontal directions, and the size of one side of the region 41h to be determined is 1/2a pixels. In Fig. 8, the region 41h to be determined is hatched. In addition, the examination image has a size of 10 pixels or more in the vertical and horizontal directions.

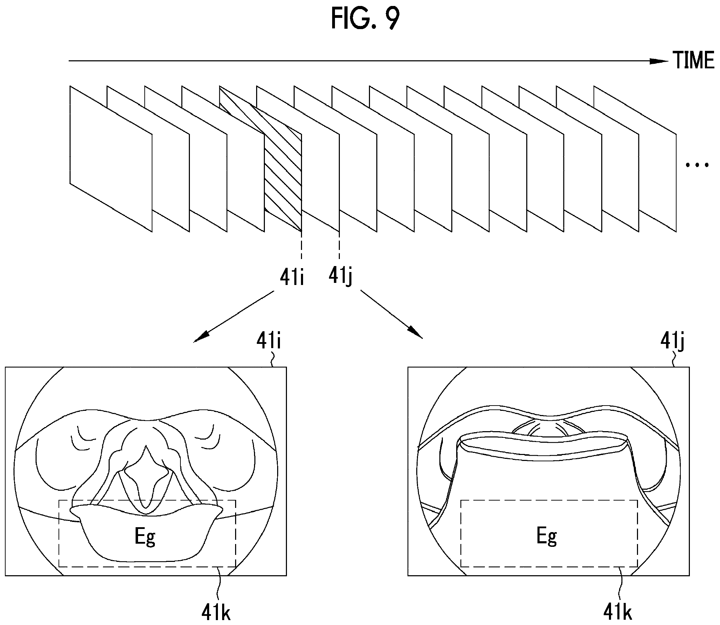

Further, an epiglottis region may be detected from the examination image and may be used as the region to be determined. For example, as illustrated in Fig. 9, in a case in which an examination image 41i and an examination image 41j are the examination images acquired in time series, an epiglottis region 41k may be detected from the examination image 41i. Then, in the examination image (for example, in the examination image 41j) acquired after the examination image 41i, the epiglottis region 41k may be used as the region to be determined. The epiglottis region 41k may be determined in a case in which the epiglottis is detected for the first time after the examination image is acquired during endoscopy or may be determined in a case in which the magnification of the endoscope 12 is changed. The above-described configuration makes it possible to classify the examination images into an image showing the swallowing state or an image showing the non-swallowing state according to the area of the overexposed region. The user can see the examination image determined to show the swallowing state to perform various kinds of diagnoses. Therefore, the examination is smoothly performed, and it is possible to prevent oversight.

In a case in which the examination image is input, it is preferable that the second determination unit 42 calculates the probability of the examination image showing the swallowing state and outputs that the examination image shows the swallowing state or the non-swallowing state. It is preferable that the second determination unit 42 includes a classifier 42a which determines whether the examination image shows the swallowing state or the non-swallowing state. The classifier 42a is a classifier that is generated by using machine learning. It is preferable to use deep learning as the machine learning. For example, it is preferable to use a deep convolutional neural network. The machine learning includes, for example, decision trees, support vector machines, random forests, regression analysis, supervised learning, semi-supervised learning, unsupervised learning, reinforcement learning, deep reinforcement learning, learning using neural networks, and generative adversarial networks in addition to the deep learning.

It is preferable that the classifier 42a is machine learning that has learned the image determined to show the swallowing state and the image determined to show the non-swallowing in advance. In addition, the classifier 42a may be machine learning using unsupervised learning or semi-unsupervised learning that automatically clusters the image showing the swallowing state and the image showing the non-swallowing.

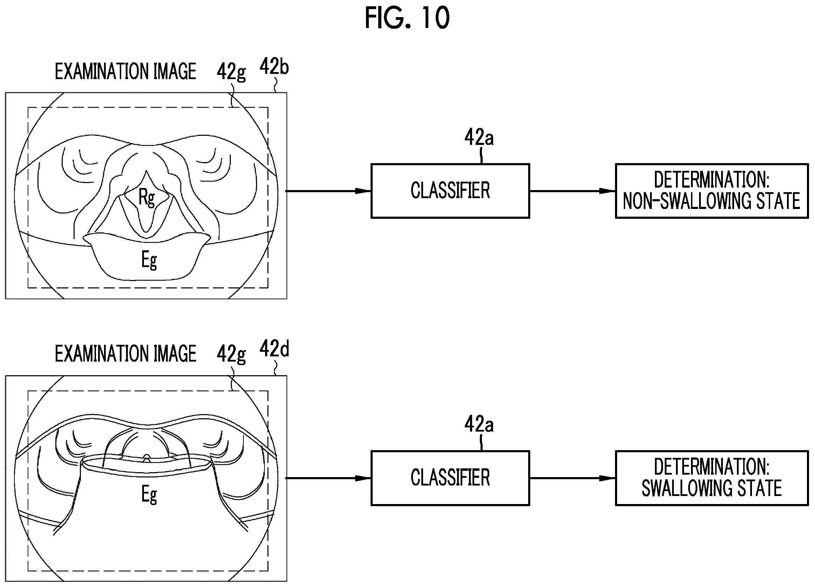

Fig. 10 illustrates a specific example of the determination of the swallowing by the second determination unit 42. In the examination images (an examination image 42b in an upper part of Fig. 10 and an examination image 42d in a lower part of Fig. 10) input to the classifier 42a, a region having a size of at least 224 pixels from the image center of the examination image of at least one frame in the vertical and horizontal directions is defined as a region 42g to be determined. A specific example in the upper part of Fig. 10 is an example using the examination image 42b in which the swallowing movement does not occur. In a case in which the examination image 42b is input to the classifier 42a, it is determined to be the image showing the non-swallowing state. A specific example in the lower part of Fig. 10 is an example using the examination image 42d in which the swallowing movement occurs. In a case in which the examination image 42d is input to the classifier 42a, it is determined to be the image showing the swallowing state.

Further, in addition to the image signal input from the imaging sensor, the examination image determined to show or not to show the swallowing state by the first determination unit 41 may be used as the examination image input to the classifier 42a. Furthermore, the first determination unit 41 may correct the determination of whether the examination image, which has been determined to show or not to show the swallowing state by the classifier 42a, shows the swallowing state or the non-swallowing state. The corrected result may be used to train the classifier 42a. The above-described configuration makes it possible to classify the examination images into the image showing the swallowing state and the image showing the non-swallowing state.

The examination image determined to show the "swallowing" state or the "non-swallowing" state by the first determination unit 41 or the second determination unit 42 may be output to the result recording unit 34. In addition, it is assumed that the swallowing is determined in real time during the examination and the determination result is displayed on an examination screen. However, the swallowing may be determined after the examination is ended. The determination may be automatically performed after the examination is ended, and the determination result may be recorded. The determination may be performed only in a case in which an instruction to perform the determination only on necessary moving images is received from a user such as a doctor. The determination of the swallowing may be performed in a case in which an image is called from a recording device 17, such as a picture archiving and communication system (PACS), an electronic medical record, or a server, and then displayed. The computer 16 may read the moving image recorded on an external recording device, such as a universal serial bus (USB) memory, and perform the determination independently of the processor device 15. The above-described configuration makes it possible to automatically determine swallowing from the examination image to support the user's diagnosis.

The examination image determined to show the "swallowing" state or the "non-swallowing" state by the first determination unit 41 or the second determination unit 42 is output to the swallowing moving image creation unit 43. The examination images output to the swallowing moving image creation unit 43 are a series of moving images which are associated with the time (examination time) when the examination images were acquired and are arranged in time series. After acquiring the examination image determined to show the swallowing state, it is preferable that the swallowing moving image creation unit 43 determines the examination images of the frames acquired for a predetermined period to show the swallowing state and outputs the examination images acquired for the predetermined period as a series of swallowing moving images.

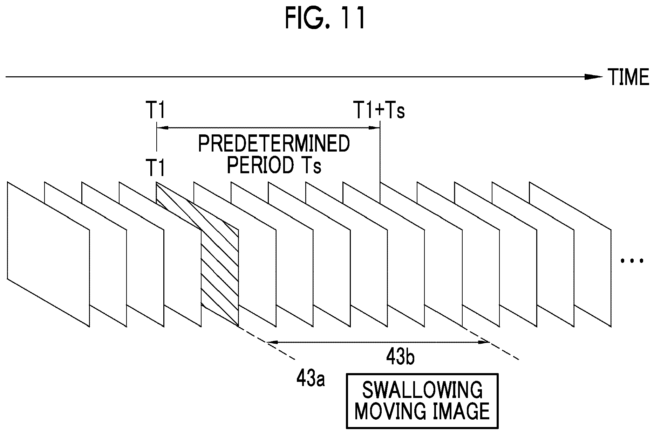

Fig. 11 illustrates a specific example of the creation of the swallowing moving image. In a case in which there is an examination image 43a determined to show the "swallowing" state by the first determination unit 41 or the second determination unit 42 among the examination images which are a series of moving images, the swallowing moving image creation unit 43 sets, as T1, the time when the examination image 43a determined to show the "swallowing" state was acquired. The examination images acquired for a period T1 + Ts from the time T1 to a predetermined period Ts are used as a swallowing moving image 43b.

It is preferable that the length of the predetermined period for which the swallowing moving image creation unit 43 creates the swallowing moving image 43b can be set to any value. Further, the length of the predetermined period may be automatically set on the basis of the time required for the swallowing operation. For example, in a case in which a 30-ml water swallowing test is performed on a healthy person, the time required for swallowing is within 5 seconds. Therefore, the predetermined period is set to 5 seconds. There are various types or amounts of objects that are swallowed by the subject in swallowing function tests, such as a repeated saliva swallowing test, a water swallowing test, and a food test. Therefore, the predetermined period may be changed for each swallowing function test.

The swallowing moving image 43b may be output in a form tagged as the swallowing moving image 43b among all the examination images (moving images). Further, the swallowing moving image 43b may be output alone. The output swallowing moving image 43b is recorded on the recording device 17 through the result recording unit 34. Among all the examination images (moving images), the swallowing moving image 43b may be tagged and recorded. Furthermore, only the swallowing moving image 43b may be recorded. In a case in which the swallowing moving image 43b is recorded alone, it is preferable to collectively record one or more swallowing moving images 43b acquired in one examination in one folder. The above-described configuration makes it possible to create a series of swallowing moving images on the basis of the examination images classified as the images showing the swallowing state or the non-swallowing state. Therefore, the user can make a diagnosis while viewing the swallowing moving image. In addition, it is possible to reduce the time and effort required to search for a swallowing part from the entire moving image. Further, it is possible to smoothly review the moving image obtained by the examination in a case in which a follow-up observation or a conference is conducted.

In this embodiment, the example in which the processor device 15 and the computer 16 are provided in the endoscope system 10 has been described. However, the invention is not limited thereto, and other medical apparatuses may be used. Further, a rigid scope or a flexible scope may be used as the endoscope 12. Furthermore, the image acquisition unit 31 and/or the control unit 30 of the endoscope system 10 may be provided in, for example, a medical image processing device that communicates with the processor device 15 and cooperates with the endoscope system 10. For example, the image acquisition unit 31 and/or the control unit 30 may be provided in a diagnosis support device that acquires the image captured by the endoscope 12 directly from the endoscope system 10 or indirectly from the PACS. Moreover, the image acquisition unit 31 and/or the control unit 30 in the endoscope system 10 may be provided in a medical service support device that is connected to various examination apparatuses, such as a first examination apparatus, a second examination apparatus,..., an N-th examination apparatus, including the endoscope system 10 through a network.

In this embodiment, the hardware structures of the processing units executing various processes, such as the control unit 30 and the central control unit (not illustrated), are the following various processors. The various processors include, for example, a central processing unit (CPU) which is a general-purpose processor executing software (programs) to function as various processing units, a programmable logic device (PLD), such as a field programmable gate array (FPGA), which is a processor whose circuit configuration can be changed after manufacture, and a dedicated electric circuit which is a processor having a dedicated circuit configuration designed to perform a specific process.

One processing unit may be configured by one of the various processors or may be configured by a combination of two or more processors of the same type or different types (for example, a combination of a plurality of FPGAs or a combination of a CPU and an FPGA). Further, a plurality of processing units may be configured by one processor. A first example of the configuration in which a plurality of processing units are configured by one processor is an aspect in which one processor is configured by a combination of one or more CPUs and software and functions as a plurality of processing units. A representative example of this aspect is a client computer or a server computer. A second example of the configuration is an aspect in which a processor that implements the functions of the entire system including a plurality of processing units using one integrated circuit (IC) chip is used. A representative example of this aspect is a system-on-chip (SoC). As described above, various processing units are configured using one or more of the various processors as a hardware structure.

In addition, specifically, an electric circuit (circuitry) obtained by combining circuit elements, such as semiconductor elements, can be used as the hardware structure of the various processors. Further, the hardware structure of the storage unit is a storage device such as a hard disc drive (HDD) or a solid state drive (SSD).

### Explanation of References

10: endoscope System
12: endoscope
12a: insertion portion
12b: operation portion
12c: bending portion
12d: tip portion
12e: angle knob
12h: still image acquisition instruction switch
12i: zoom operation portion
14: light source device
15: processor device
16: computer
17: recording device
18: display
19: user interface
20: light source
22: light source control unit
30: control unit
31: image acquisition unit
32: display control unit
33: image input unit
34: result recording unit
40: swallowing determination unit
41: first determination unit
41a, 41c: grayscale image
41b, 41d: binarized image as example of image showing non-swallowing state
41e: low pixel value region
41f: high pixel value region
41g: image center
41h, 42g: region to be determined
41i, 41j, 42b, 42d, 43a, 100: examination image
41k: epiglottis region
42: second determination unit
42a: classifier
43: swallowing moving image creation unit
43b: swallowing moving image
Es: esophagus
Eg: epiglottis
Ev: epiglottic vallecula
F: food
Rg: rima glottidis
Ps: pyriform sinus
Sp: soft palate
To: tongue
Tr: trachea

## Claims

1. An endoscope system that illuminates an object and captures light from the object, the endoscope system comprising:
a control processor (15) configured to:
acquire an examination image; and
determine whether the examination image shows a swallowing state or a non-swallowing state.

2. The endoscope system according to claim 1,
wherein the control processor is configured to:
detect a high pixel value region from the examination image; and
determine that the examination image shows the swallowing state in a case in which an area of the high pixel value region is equal to or greater than a first threshold value.

3. The endoscope system according to claim 2,
wherein the control processor is configured to:
perform grayscale conversion on the examination image to obtain a grayscale image; and
perform a binarization process for obtaining the high pixel value region in a case in which a density value of a pixel of the grayscale image is equal to or greater than a second threshold value.

4. The endoscope system according to claim 2 or 3,
wherein the control processor is configured to decide a region to be determined from the examination image and detect the high pixel value region from the region to be determined.

5. The endoscope system according to claim 4,
wherein the region to be determined is a region in a range which has at least 10 pixels or more from an image center of the examination image in a vertical direction and a horizontal direction and in which a size of one side of the region to be determined is equal to or less than half a size of the smaller of vertical and horizontal sides of the examination image.

6. The endoscope system according to claim 4,
wherein the control processor is configured to detect an epiglottis region from the examination image and use the epiglottis region as the region to be determined.

7. The endoscope system according to any one of claims 1 to 6,
wherein the control processor is configured to input the examination image to a classifier and output the examination image determined to show the swallowing state or the non-swallowing state.

8. The endoscope system according to claim 7,
wherein the classifier is trained with an image determined to show the swallowing state or the non-swallowing state.

9. The endoscope system according to any one of claims 1 to 8,
wherein the control processor is configured to determine, after acquiring the examination image determined to show the swallowing state, that the examination images of frames acquired for a predetermined period show the swallowing state and output the examination images acquired for the predetermined period as a swallowing moving image.

10. The endoscope system according to claim 9,
wherein the control processor is configured to record the examination images acquired for the predetermined period as the swallowing moving image.

11. The endoscope system according to claim 9 or 10,
wherein the control processor is configured to tag the swallowing moving image among the examination images acquired for the predetermined period and output the tagged swallowing moving image.

12. The endoscope system according to any one of claims 9 to 11,
wherein the control processor is configured to tag the swallowing moving image among the examination images acquired for the predetermined period and record the tagged swallowing moving image.

13. The endoscope system according to any one of claims 9 to 12,
wherein the predetermined period is settable to any value.

14. The endoscope system according to any one of claims 9 to 13,
wherein the predetermined period is automatically set on the basis of a time required for a swallowing movement.

15. A method for operating an endoscope system (10) that illuminates an object, captures light from the object, and includes a control processor (15), the method comprising:
a step of causing the control processor to acquire an examination image; and
a step of causing the control processor to determine whether the examination image shows a swallowing state or a non-swallowing state.
